Europäisches Patentamt

European Patent Office

Office européen des brevets

(11) Publication number: **0 566 109 A1**

## EUROPEAN PATENT APPLICATION

(21) Application number: **93106115.4**

(22) Date of filing: **15.04.93**

(51) Int. Cl.5: **C12Q 1/04**, C12Q 1/28, G01N 1/30

(30) Priority: **17.04.92 JP 125641/92**

(43) Date of publication of application: **20.10.93 Bulletin 93/42**

(84) Designated Contracting States: **DE FR GB**

(71) Applicant: **KING BREWERY CO., Ltd.**
**321, Takokusa,**
**Inami-cho**
**Kako-gun, Hyogo-ken(JP)**
Applicant: **Kurashiki Boseki Kabushiki Kaisha**
**7-1, Hommachi**
**Kurashiki-shi Okayama-ken(JP)**

(72) Inventor: **Yamashoji, Shiro**
**514, 9-50, Kaigan-dori,**
**Tarumi-ku**
**Kobe-shi, Hyogo-ken(JP)**
Inventor: **Nishimoto, Fukiko**
**1178-54, Rokubuichi,**
**Inami-cho**
**Kako-gun, Hyogo-ken(JP)**

(74) Representative: **von Kreisler, Alek et al**
**Patentanwälte**
**von Kreisler-Selting-Werner,**
**Postfach 10 22 41,**
**Bahnhofsvorplatz 1**
**D-50462 Köln (DE)**

(54) **Colorimetric assay for the determination of density or activity of viable cells.**

(57) A convenient and rapid assay for activity and density of living cells in many samples is provided. That is, a oxidized type quinone such as 1,4-naphthoquinone, 2-methyl-1,4-naphthoquinone or tetramethyl-p-benzoquinone is reacted with a system to be determined. The Hydrogen peroxide thus produced is indirectly reacted with the colorimetric reagent by using enzyme or directly with the colorimetric reagent, the intensity of the color development thus caused is determined with a spectro phtometer(a colorimeter). A proper enzyme of the acceptor of hydrogen peroxide is peroxidase, indirect color reagent is sodium salt of N-(carboxymethylaminocarbonyl)-4,4'-bis(dimethylamino)-diphenylamine or leucocrystal violet are suitable for indirect colometric reagent, and direct colorimetric reagent is cytochrome c peroxidase.

[Fig. 1]

Cell density($\times 10^3$ cells/ml)

Technical Field

This invention relates to method for assaying the activity or density of living(viable) cells or of such cells in living tissue or organ rapidly and exactly.

Background of the Invention

Heretofore, biological tests by experimental animals have mainly done for estimation of the safety of pharmaceuticals, cosmetics or foods during their study and development. However, recently, it has been taken notice to estimate their safety by substitutes for animal tests in views of its cost reduction,convenience and rapidity,and various cytotoxicity assays have been developed with animal cell culture. As the typical methods among the developed assays, neutral red inclusion assay and MTT reduction assay are well known. For example, neutral red-intake by lysosome or the amount of insoluble product of MTT reduced by mitochondria in viable cells, respectively is automatically and quantitatively determined by a microplate reader spectrophotometrically. The viability of the tested cells is measured according to the amounts of neutral red or insoluble formazan accumulated in viable cells.

But, this assay requires 3 to 5 hours' long incubation, and the activities of the tested cells might be altered during such long incubating time for sufficient accumulation of the dye into cells. Thus, this known colorimetry is not available to trace prompt change in cytotoxity which might be arisen by minute unit.

So, present inventors have invented and proposed novel chemiluminescent assay as Japanese patent laid-open 160499/1989 as more rapid assay for biological determination of viable cells by which the density or activity of viable cells are changed within few minutes from the start. This chemiluminescent assay is very rapid way for the above purpose because the quantity of hydrogen peroxide ($H_2O_2$) generated can be determined only within 5 seconds after the incubation of the cells with an oxidized type quinone, for several minutes. There is a good proportional correlation between the quantity of hydrogen peroxide and activity or density of viable cells.

But, in this chemiluminescent assay, the reaction is inhibited by various ions, especially halogen ion, contained in the culture media for multipication or maintenance. Therefore, in order to increase accuracy of the assay, it is necessary to satisfy the following conditions:---

① To prepare plenty numbers of sample cells in order to level up the concentration of hydrogen peroxide (more than $10^{-6}$ M which is the threshold for determination.

② To select an appropriate culture medium which hardly inhibites chemiluminescent reaction.

③ To prepare special instruments for measuring chemiluminescence.

Summary of the Invention

Thus, the object of present invention is to provide a rapid and exact assay for the determination of density (the numbers of cells existing in a constant volume) or activity of viable (living)cells *per se* or of such cells in living tissue or organ for biological studies by common microplate reader.

Brief Description of the Drawings

Fig.1 is a graph showing the correlation of density between animal cells and hydrogen peroxide production, according to present invention.

Fig.2 is a graph showing the inhibitory action of various surfactants against animal cells, according to present invention.

Fig.3 is a graph showing the inhibitory action of various surfactants against animal cells, according to chemiluminescent assay and neutral red intake assay.

Fig.4 is a flow chart of standard process for the assay according to the present invention.

Detailed Explanation of the Invention

(Gist of the invention)

Now, the present invention exists in a colorimetric assay for the determination of density or activity of viable cells which comprises incubating viable cells in the presence of an oxidized type quinone to produce hydrogen peroxide, reacting the hydrogen peroxide, directly or intermittent with an enzyme which is an acceptor of said hydrogen peroxide with a color reagent and determining the intensity of the coloring caused by a spectrophotometer.

(Principle of the Invention)

Namely, it has been believed that in cell membrane and cytoplasm, there are oxido-reducutases which reduce the oxidzed products in cells, in the aid of reduced type enzymes such as nicotinamide adenin dinucleotide(NADH)and its phosphate(NADPH). Especially, the presence of NAD(P)H:quinone oxido-reductase is well-known. The principle of the present invention is to spectrologically determine hydrogen peroxide in a small reaction system, based on the our foregoing invention wherein being disclosed that quinone-compounds, represented by 2-methyl-1,4-naphthoqininoe(menadione) react with viable cells and hydrogen peroxide is accumulated in the incubated culture medium. And, the present invention includes two modes of assay for its embodiments as follows:---

In one assaying mode(A)according to the present invention, the density or activity of the viable cells to be tested is spectrophotometrically determined by the intensity of the color formed by the reaction of hydrogen peroxide which was formed by indirect reaction of qininones and accumulated outside the cell system with a color reagent intermittent with an enzyme, an acceptor of hydrogen peroxide, such as peroxidase. In another assaying mode(B), the density or activity of the viable cells to be tested is spectrophotometrically determined by the intensity of the color formed by the reaction of hydrogen peroxide which was formed by direct reaction of quinones and accumulated outside the cell system with a color reagent such as cytochrome c peroxidase.

(Enzyme)

It is one of characteristic of our invention that an enzymatic reaction is applied to, in order to detect very small amount of hydrogen peroxide formed from a reaction system which is comparably smaller amount and lower density of cells as compared with prior chemiluminescent assay. There are many kinds of oxido-reductases who concerns with hydrogen peroxide, however, in the purpose of the present invention, it is prefer red an oxidative enzyme which colors oxido-reductase, color reagent etc. by oxidation, i.e., peroxidase. Peroxidase on the market is, for instance, three valent's Fe-porphyrin protein extracted from horseradish or milk etc.

(Color reagent)

As the indirect color reagent according to the above mode A, it can be illustrated strong chromgenic substances such as o-phenylenediamine, 3,3'-diaminobenzidine, 2,2'-azino-di(3-ethylbenzthiazoline sulfonic acid), leucocrystal violet, sodium salt of N-carboxymethylaminocarbonyl-4,4'-bis(dimethylamino )-diphenylamine(abbreviation:DA-64) or sodium salt of 10-(carboxyethylaminocarbonyl)-3,7-bis-(dimethylamino)-phenothiazine etc., but, the latter two compounds are most preferable in views of the sensitivity and solubility to substrates. Besides, any colorreagent which reacts with quinones so as to form coloring or decoloring is not preferred since the colorimetric system according to the present invention involves quinones such as menadione.

As the direct color reagent according to the above mode B, a reagent which directly reacts with hydrogen peroxide and produces a dye without intermittence of any oxido-reductase Such as cytochrome c peroxidase is used. This enzyme instantly forms a stable complex. The optical absorbance of this complex( $\lambda_{max}$ 419 nm) is different from that of the enzyme itself( $\lambda_{max}$ 408 nm) so that the concentration of produced hydrogen peroxide can be obtained by determining the difference between the both absorbances. Here, the mole absorption constant of said difference $\Delta \epsilon$ is 50,000 $1 \cdot mol^{-1} \cdot cm^{-1}$.

(Determination)

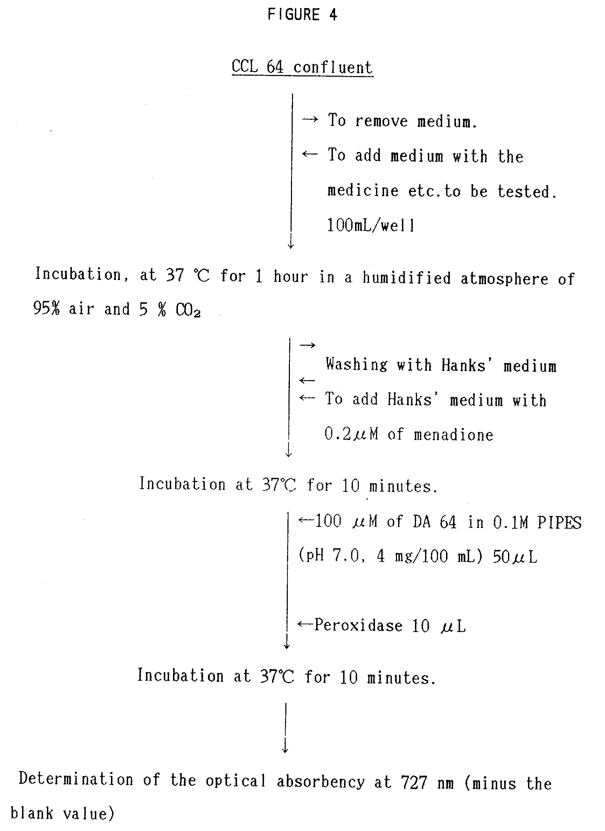

At first, generally, the numbers of cultured cells in the medium is adjusted to $10^3$ to $10^7$ cell/m$\ell$ and then, a destined amount of said medium is poured into each well of 96-well microplate. Thereafter, the cells(in the wells) are incubated for a destined time after the addition of a destined amount of a quinone compound. To the incubated there are further added a destined amount of PIPE (Piperazine-N,N'-bis-(2-ethane sulfonic acid)) buffer solution containing a destined concentration of a color reagent and then PIPE buffer solution containing a destined amount of peroxidase, respectively, followed by further incubation for a destined period. Next,the optical absorbance of the sample together with that of the blank (control, before colored) is measured under a destined wave length (it is 727 nm in the DA-64, above) by a spectrophotometer(colorimeter), respectively, and finally, the amount of hydrogen peroxide generated is determined on the basis of the value of the remainder obtained by reducing the blank value from that of the sample according to forehand determination line. Fig.4 shows a standard process according for the above assay.

(Action)

The assay according to the present invention uses a coloring reaction of a color reagent induced by the oxidative action of an enzyme which is a receptor of hydrogen peroxide, or a coloring reaction between a color reagent by said hydrogen peroxide and a color reagent. Therefore, this assay is applicable to quantitative analysis of hydrogen peroxide from minute amount of sample so that it is applicable to common 96-well microplate for simultaneous analysis for many samples. Furthermore, since this assay is not injured by haolgen in the substrate to be tested, it is applied for direct assay of the culture as has descrived in the paragraph "Determination", above.

EXAMPLES

The present invention is further embodied by the following Examples but they are intended to be for the illustration and not for the limitation.

Example 1 (Measurement of the numbers of viable cells)

(1) Operation

① Mink lung cells(Mv1Lu:ATCC CCL64) were cultured under conditions of 37°C, 5 %$CO_2$, saturated vapor. The medium was high glucose-Dulbecco-modified Eagle's medium containing 0.4 % glutamine, 0.4 % penicillin and streptomycin, and 10 % bovine serum.

② The cells were treated with trypsin under a subconfluent state and centrifuged for the collection of free cells. Then medium was replaced by Hank's medium or MEM medium.

③ The cells collected were diluted to about $10^3$ to $10^7$ c ells/ mℓ with Hank's or MEM medium, and then were poured 25μℓ each into each well of 96-well microplate.

④ 25 μℓ of Hank's or MEM medium containing 0.4 mM of menadione was added to cell suspension of each well and the microplate was incubated at 37°C for 10 minutes. To the wells incubated, there were added 50 μℓ /well of PIPES buffer solution containing 100 μM of DA-64 and 10 μℓ/well PIPES buffer solution containing peroxidase(125 U/ mℓ)to each well by turn, and thenre-incubated at 37°C for 10 minutes.

⑤ The optical absorbance of each well was measured at 727 nm and compared that of the control in which no cell was present but was treated as the same as the sample wherein cells to be tested being inclusive to obtain the reduction value in order to obtain the amount of hydrogen peroxide formed in each well according to forehand determination line.

(2)Result and Discussion

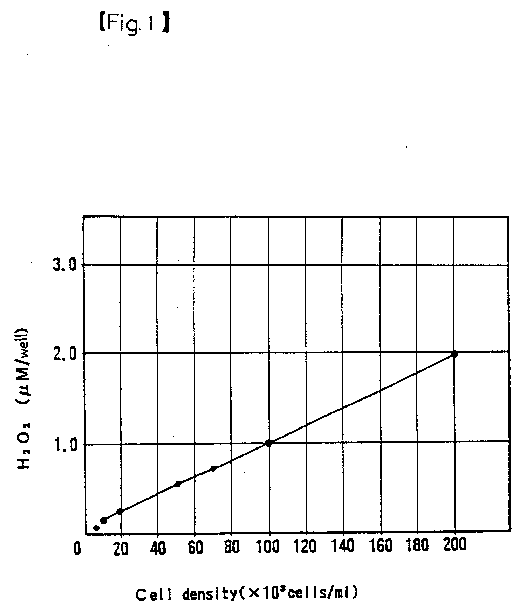

As shown by Figure 1, there is relationship of direct proportion between the number of viable cells and the amount of hydrogen peroxide produced. The measurable range of viable cells is $10^4$ to $2 \times 10^5$ cells/mℓ, and in 96-well microplate there could be measured of the range from $2.5 \times 10^2$ to $5 \times 10^3$ cells/well. In this example,the assay required 10 minutes for incubation of viable cells and menadione, and further 10 minutes for analysis of hydrogen peroxide, that is, 20 minutes totally.

Contrary to this, in our prior chemiluminescent assay, the direct time necessitated for photometry is as short as 5 seconds, however, it is not applicable to 96 well microplate in view of sensitivity of colorimetric instrument at present. In addition, said prior art is not applicable for cells in culture medium, therefore, it is needed to substitutean appropriate medium not containing halogen ion who might influence to the real activity of the viable cells to be tested.

In consideration of these, the present assay seems practically better for the measurement of viable cells with the most conventional 96-well microplate.

Example 2 and Comparisons 1 and 2(Measurement of cytotoxity by present invention and controls)

Cytotoxity against animal cellls was comparably estimated between three methods of the present assay, chemiluminescent assay(according to our prior method) and neutral red intake method.

Materials(Tested surfactants etc.)

| Abbreviations et al. | Chemical Name |
|---|---|
| BC :<br>ABAC :<br>Tween 20 :<br>glycerol SDS | Benzethonium chloride<br>Alkylbenzyl dimethylammonium chloride<br>Polyoxyethylene sorbitan monolaurate<br>Sodium dodecylsulfate |

Operation(Example 2)

(1-1) Invented assay (enzymatic assay)

① 2 x $10^4$ cells of mink lung cell(Mv1Lu:ATCC CCL64) were added into each well of a 96-well microplate and incubated for 3 days,at 37°C in a humidified atmosphere of 95% air and 5 % $CO_2$. The culture medium was same as Example 1.

② Next, the medium was replaced by another medium which containing destined concentration of several surfactants under a confluent condition and further incubated for 1 hour.

③ After the incubation, the cells were repeatedly washed with Hank's medium in order to remove the surfactant off.Then, to each well, there were added 50µℓ/well of Hank's medium containing 0.2 mM of menadione and then incubated for 10 minutes at 37 °C followed by further addition of 50 µℓ/wel of PIPES buffer solution containing 100 µM of DA-64 and 10µℓ/well of PIPES buffer solution containing 125U/ mℓ of peroxidase, and further incubated at 37 °C for 10 minutes.

④ Thereafter, the optical absorbance of each well was measured at 727nm and thus the amount of hydrogen peroxide was obtained by reducing blank value from the control not containing cells based on the examination line.

⑤ The activity of the tested cells will be estimated as the percentage of the amount of hydrogen peroxide from surfactant-treated cells to that from non-treated cells.

(1-2) Comparison 1 (Chemiluminescent assay)

① 1.5 x $10^5$ cells of mink lung cell(Mv1Lu:ATCC CCL64) were added into each well of a 24-well microplate and incubated for 3 days, at 37°C in a humidified atmosphere of 95% air and 5 % $CO_2$. The culturemedium was same as Example 1.

② Then, the medium was subsituted with another medium which contained destined concentration of several surfactants under a confluent condition and further incubated for 1 hour.

③ After the incubation, the cells were repeatedly washed with MEM- α (the abbreviation of Minimum Essential Medium Eagle Alpha Modification) to remove the surfactant off, and then 500 µL of MEM- α were added to each well.

④ Succeedingly, 5µℓ /well of 20 mM menadione solution were added to the well, and then further incubated at 37 °C for 10 minutes. The further addition of 500 µℓ/well of reagent comprising of TDPO (bis[4-nitro-2-(3,6,9-trioxadecyloxycarbonyl)phenyl]oxalate) and pyrene caused the chemiluminescence whose intensity was measured for 5 seconds.

⑤ The amount of hydrogen peroxide in the sample is, then, determined by substracting blank value from the control wherein the cells were not contained according to destined examination line. The activity of the tested cells will be estimated as the percentage of the amount of hydrogen peroxidefrom surfactant-treated cells to that from non-treated cells.

(1-3) Comparison 2 (Neutral red intake assay)

① 2 x $10^4$ cells of mink lung cell(Mv1Lu:ATCC CCL64) were added into each well of a 96-well microplate and incubated for 3 days,at 37°C in a saturated With 5 % $CO_2$. The culture medium was same as Example 1.

② Then, the medium was replaced by another medium containing destined concentration of several surfactants under a confluent condition and further incubated for 1 hour.

③ Next,the cells were repeatedly washed to remove the surfactant off, and then incubated at 37°C for 3 hours in a saturated With 5 % $CO_2$ with the addition of 100$\mu\ell$/well of NR(neutral red) to each well.

④ Thereafter, the NR solution was removed, and the cells were fixed with a fixing solution (10 % formalin) of 100 $\mu\ell$ to each well,and then,allowed to stand for 1 minute at a room temperature.

⑤ Then,the fixing solution in each well was replaced with 100$\mu\ell$ of an extracting solution(acidic isopropanol) and the plate was incubated for 20 minutes at a room temperature.

⑥ After slight agitation was given, optical absorbance at 550 nm to calculate the activity of viable cells in comparison with that of the control from non-treated cells.

(2) Result and discussion

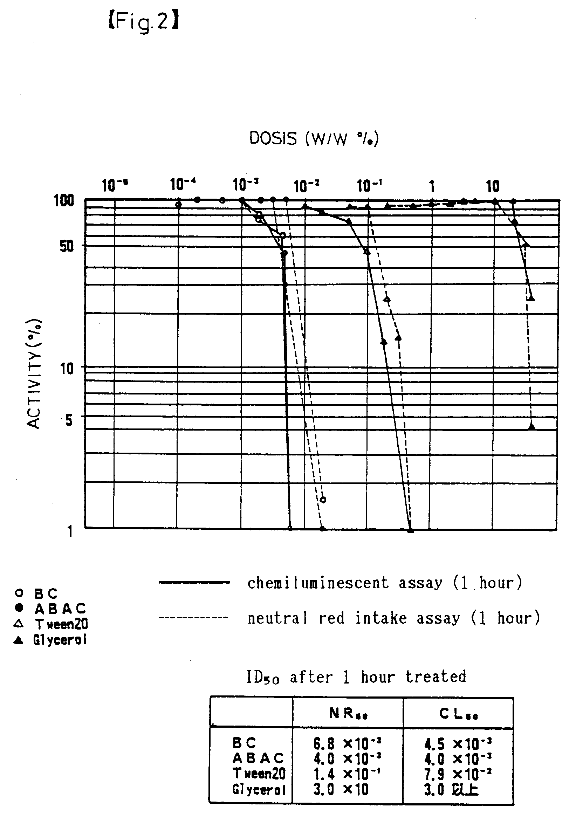

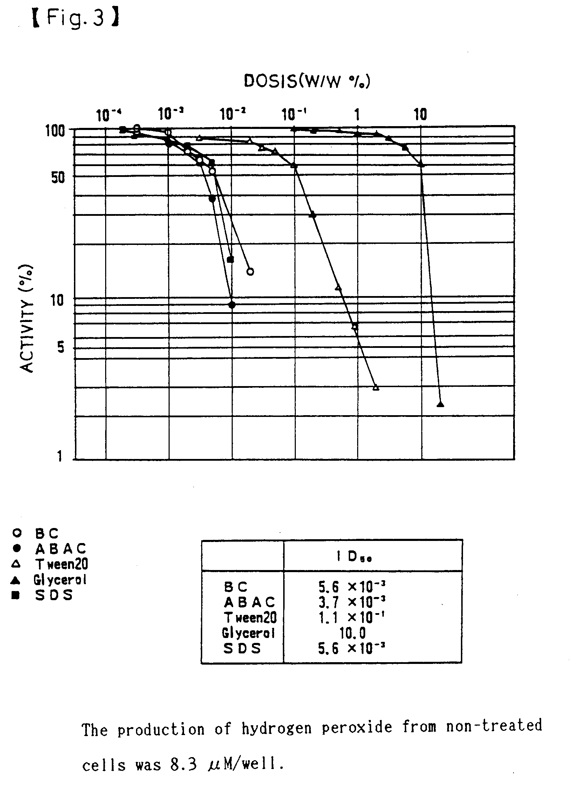

As shown in Fig.2 and Fig.3 attached, there could be found no difference between the curves of the inhibition. Thus,it is clear that the invented assay will be applicable to determination of cytotoxity likewise prior arts.

Example 3 (Applicability of quinone)

We have further studied the applicability of many quinone compounds to the assay of the activity of animal cells, CCL64, PC-3 and NIH 3T3. As the results, it has found that, as shown by the following Table-1, three quinones, 1,4-naphtho-quinone, 2- methyl-1,4-naphthoquinone(menadione) and tetramethyle-parabenzoquinone were effective to any kind of cells, but 1,2-naphthoquinone, 2-hydroxy-1,4-naphthoquinone, 1,5-di-aminoanth raquinone and p-benzoquinone were ineffective to form measurable amount of hydrogen peroxide to any tested strain. On the other side, 2-hydroxy-1,4-naphthoquinone, disodium anthraquinone-2,6-disulfonic acid, anthraquinone, chloranyl and chloranylic acid did not form measurable amount of hydrogen peroxide and did not produce hydrogen peroxide due to the strain of cells. This suggests an relationship between the enzymatic action of the inherent cell and chemical structure of quinones.

# EP 0 566 109 A1

Table-1

| Amout of H₂O₂ produced* / Chemical name of Quinones | Kind of animal cells | | | Chemical formulae |
|---|---|---|---|---|
| | CCL-64 | PC-3 | NIH 3T3 | |
| 1,2-Naphtho-quinone | — | — | — | |
| 1,4-Naphtho-quinone | 2.7 (±0.54) | 15.3 (±2.5) | 9.3 (±0.85) | |
| 2-Methyl-1,4-naphthoquinone | 3.98 (±0.55) | 27.3 (±2.5) | 13.8 (±2.5) | |
| 2-Hydroxy-1,4-naphthoquinone | — | — | — | |
| 1,5-Diamino-anthraquinone | — | — | — | |
| Anthraquinone-2,6-disulfonic acid dinatrium | — | 2.8 (±0.4) | — | |
| Anthraquinone | — | 0.99 (±0.01) | — | |
| p-Benzoquinone | — | — | — | |
| Chloranyl | — | — | — | |
| Chloranylic acid | — | 1.3 (±0.01) | — | |
| Tetramethyl-p-benzoquinone | 0.6 (±0.26) | 44.9 (±6.0) | 6.8 (±0.8) | |

\* The amount of the hydrogen peroxide produced (μM/well) is given as the average of 3 specimens (the numerals closed by round brackets show the standard deviation)
— : None of hydrogen peroxide dectable.

In this experiment, the tested cells were incubated in a 24-well microplate, then the culture medium in each well was replaced by 500 μℓ of MEM-α when the cell became confluent, and 20 μL of respective quinone compound was added to each well followed by the incubation at 37 °C for 10 minutes. Then, the amount of hydrogen peroxide produced was assayed as in Example 1.

7

Example 4

CL$_{50}$ of five substances was measured according to the method described in Example 2 (See Figuire 2.). Adversely, DS$_{20}$ of the same substances was calculated from Draise's test according to Torishima's method (AATEX Vo.l 1, page 20-26,1990 ). As shown by Table-2, below, CL$_{50}$ showed good proportion to Draize Score rank (DS rank) simultaneously calculated.

Table 2

|  | CL$_{50}$ (w/w %) | DS$_{20}$ (w/w %) | DS rank |
|---|---|---|---|
| BC | $4.6 \times 10^{-3}$ | 1.5 | Severe |
| ABAC | $3.8 \times 10^{-3}$ | 2.0 | Severe |
| Tween 20 | $76 \times 10^{-3}$ | 7100 | Non |
| Glycerol | $24800 \times 10^{-3}$ | 7100 | Non |
| SDS | $3.8 \times 10^{-3}$ | 4.5 | Moderate |

(Application of the present invention)

As explained and embodied hereinabove, the present invention provided convenient and prompt assay of many samples for the density or the activity of viable cells by microplate reader. Thus, it is not only useful for the cytotoxicity test of pharmaceuticals and foods but also for general biological studies.

**Claims**

1. A colorimetric assay for the determination of density or activity of viable cells which comprises incubating viable cells in the presence of an oxidized type quinone, incubating the produced hydrogen peroxide with an enzyme and the color reagent indirectly reacting with hydrogen peroxide, or with a colorimetric reagent directly reacting with hydrogen peroxide and measuring the intensity of the color development with a spectrophotometer.

2. The assay according to Claims 1, wherein the enzyme used for color development is peroxidase.

3. The assay according to Claims 1, wherein the oxidized type quinone is 1,4-naphthoquinone, 2-methyl-1,4-naphthoquinone (menadione) or tetramethyl-p-benzoquinone.

4. The assay according to Claims 1, wherein the colorimetric reagent which is coexisted with the enzyme is sodium salt of N-(carboxymethyaminocarbonyl)-4,4'-bis(dimethylamino)-diphenyl amine or leucocrystal violet.

5. The assay according to Claims 1, wherein the colorimetric reagent which is reacted with the hydrogen peroxide produced is cytochrome c peroxidase.

【Fig. 1】

C e l l   d e n s i t y ( × 10³ c e l l s / m l )

【Fig.2】

DOSIS (W/W %)

ACTIVITY(%)

| | NR$_{50}$ | CL$_{50}$ |
|---|---|---|
| B C | $6.8 \times 10^{-3}$ | $4.5 \times 10^{-3}$ |
| A B A C | $4.0 \times 10^{-3}$ | $4.0 \times 10^{-3}$ |
| T ween20 | $1.4 \times 10^{-1}$ | $7.9 \times 10^{-2}$ |
| Glycerol | $3.0 \times 10$ | $3.0$ 以上 |

ID$_{50}$ after 1 hour treated

○ B C
● A B A C
△ Tween20
▲ Glycerol

———— chemiluminescent assay (1 hour)

------------ neutral red intake assay (1 hour)

【Fig.3】

DOSIS(W/W %)

| | ID$_{50}$ |
|---|---|
| BC | $5.6 \times 10^{-3}$ |
| ABAC | $3.7 \times 10^{-3}$ |
| Tween20 | $1.1 \times 10^{-1}$ |
| Glycerol | 10.0 |
| SDS | $5.6 \times 10^{-3}$ |

O  BC
●  ABAC
△  Tween20
▲  Glycerol
■  SDS

The production of hydrogen peroxide from non-treated cells was 8.3 $\mu$M/well.

FIGURE 4

<u>CCL 64 confluent</u>

→ To remove medium.

← To add medium with the
  medicine etc.to be tested.
  100mL/well

Incubation, at 37 ℃ for 1 hour in a humidified atmosphere of 95% air and 5 % $CO_2$

→
   Washing with Hanks' medium
←
← To add Hanks' medium with
  $0.2\mu M$ of menadione

Incubation at 37℃ for 10 minutes.

←100 $\mu M$ of DA 64 in 0.1M PIPES
(pH 7.0, 4 mg/100 mL) $50\mu L$

←Peroxidase 10 $\mu L$

Incubation at 37℃ for 10 minutes.

Determination of the optical absorbency at 727 nm (minus the blank value)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-5 093 238 (S.YAMASHOJI ET AL.)<br>* the whole document * | 1,3 | C12Q1/04<br>C12Q1/28<br>G01N1/30 |
| D | & JP-A-1 160 499 (...) | | |
| X | PATENT ABSTRACTS OF JAPAN<br>vol. 016, no. 001 (C-0899)7 January  1992<br>& JP-A-32 28 696 ( KING JOZO KK ) 9 October 1991<br>* abstract * | 1,3 | |
| A | PATENT ABSTRACTS OF JAPAN<br>vol. 007, no. 066 (C-157)18 March 1983<br>& JP-A-58 000 898 ( SHINOTESUTO KENKYUSHO KK ) 6 January  1983<br>* abstract * | 1,2 | |
| A | EP-A-0 251 297 (WAKO PURE CHEMICAL INDUSTRIES, LTD.)<br>* page 1 - page 5 *<br>* page 18, line 39 - page 19, line 12 *<br>* page 6; table 1 * | 1,4 | |
| A | JOURNAL OF CHROMATOGRAPHY<br>vol. 216, 1981, AMSTERDAM NL<br>pages 413 - 416<br>W.M.DRAPER ET AL.<br>* abstract * | 1,4 | TECHNICAL FIELDS SEARCHED (Int. Cl.5)<br><br>C12Q |
| A | DE-A-3 342 977 (B.SCHÖBEL)<br>* the whole document * | 1,5 | |
| A | EP-A-0 190 740 (EASTMAN KODAK COMPANY)<br>* page 5, line 8 - page 13, line 11 *<br>* page 18, line 9 - page 19, line 15 * | 1,3 | |

The present search report has been drawn up for all claims

| Place of search | Date of completion of the search | Examiner |
|---|---|---|
| BERLIN | 25 JUNE 1993 | DE KOK A.J. |

EPO FORM 1503 03.82 (P0401)